# EUROPEAN PATENT SPECIFICATION

(11) **EP 1 790 276 B1**
(45) Date of publication and mention of the grant of the patent: **02.01.2013**
(21) Application number: 05766138.1
(22) Date of filing: 25.07.2005
(51) Int. Cl.: A61B 1/00

(54) **ENDOSCOPE, AND PRODUCTION METHOD AND REPAIR METHOD FOR THE SAME**
ENDOSKOP UND HERSTELLUNGSVERFAHREN UND REPARATURVERFAHREN DAFÜR
ENDOSCOPE, SA METHODE DE PRODUCTION ET SA METHODE DE REPARATION

(30) Priority: 26.07.2004 JP 2004217880
(43) Date of publication of application: 30.05.2007
(73) Proprietor: Olympus Corporation, Tokyo 151-0072 (JP)
(72) Inventor: MIYAGI, Masaaki, 1920032 (JP); MORIYAMA, Hiroki, 1960032 (JP); TAKASE, Seisuke, 1920916 (JP)
(74) Representative: von Hellfeld, Axel
(86) International application number: PCT/JP2005/013601
(87) International publication number: WO 2006/011460

(56) References cited:
- JP-A- 4 326 318
- JP-A- 4 326 318
- JP-U- 59 094 317
- US-A- 3 643 653
- US-A- 4 784 144
- US-A1- 2002 188 177

## Description

### TECHNICAL FIELD

The present invention relates to an endoscope having an illumination lens which is located at a distal end of an insertion portion and used in illuminating a test region, and methods of producing and repairing thereof.

### BACKGROUND ART

Conventionally, as is well known, the endoscope is widely used in the medical field and the like. In the endoscope, a long and thin insertion portion can be inserted into a body cavity to observe an organ in the body cavity and, if needed, various treatments can be performed using a treatment instrument inserted into an insertion channel of the treatment instrument.

A bendable portion and a distal end portion are provided at the distal end of the insertion portion, and an observation direction of an objective lens of an observation optical system arranged in the distal end portion can be changed by operating an operation unit of the endoscope to bend a bendable portion.

In the distal end portion, an illumination lens is also arranged to illuminate the test region (for example, an observation region in the body cavity). Particularly, the illumination lens is fixed to a distal end portion main body and a cover member for covering the distal end portion, and a distal end face of the illumination lens is exposed in an opening formed in the cover member. The illumination lens may be fitted in and fixed to a frame which is fixed to the distal end portion main body and cover member. A rear end face of the illumination lens abuts on or comes close to the distal end face of a long and thin light guide arranged in an endoscope insertion portion.

The rear end face of the light guide is configured such that the illumination light is supplied from a light source device. Therefore, the illumination light is supplied from the light source device to the light guide and output from the light guide, and the illumination lens outputs the illumination light toward the inside of the body cavity.

A bonding agent is usually used to fix the illumination lens to the cover member and distal end portion main body because of easy fixing. In addition to the bonding agent, there is also proposed a configuration in which shapes of the illumination lens and cover member are devised to fix the illumination lens more strongly.

For example, Patent Document 1 proposes a configuration, wherein the illumination lens is formed in a tapered shape by forming a diameter of the distal end portion smaller than a diameter of the rear end portion, the illumination lens is fitted in the cover-member opening whose diameter is formed smaller than the diameter of the rear end portion of the illumination lens, an outer periphery of the rear end portion of the illumination lens is fixed to a distal end portion main body with the bonding agent, and thereby the illumination lens is fixed to the cover member and distal end portion main body.

According to the above configuration, movement of illumination lens is regulated in an insertion axis direction by causing the tapered peripheral surface of the illumination lens on the inner periphery of the opening. Therefore, in the configuration proposed in Patent Document 1, the illumination lens can be fixed more strongly to the distal end portion main body and opening provided in the cover member, compared to the case where the illumination lens is fixed only with the bonding agent.
Patent Document 1: Japanese Patent Application Laid-Open No. 2002-85326
Document JP 04 326318 A concerns an endoscope having an illuminating lens fixed to a lens frame and a light guide fixed to the rear end side of the illumination lens.

### DISCLOSURE OF INVENTION

### PROBLEM TO BE SOLVED BY THE INVENTION

In the case where a lens defect such as chipping or flaws is generated in the illumination lens, it is necessary to replace the illumination lenses. In this case, it is difficult to replace the illumination lens with new one while the lens shape is maintained, because the illumination lens is fixed to the opening and the distal end portion main body with the bonding agent as described above. Therefore, in replacing the illumination lens fixed with the bonding agent, there is known a technique, wherein a bonded portion of the illumination lens is removed by cutting the illumination lens using a cutting tool such as a drill and the illumination lens is taken out from the distal end portion.

However, as described above, the distal end face of the light guide abuts on or comes close to the rear end face of the illumination lens. Furthermore, in Patent Document 1, the outer periphery of the rear end portion of the illumination lens is bonded and fixed to the distal end portion main body.

Therefore, in removing the bonded portion with the drill, sometimes there is generated a problem that the distal end face of the light guide is damaged from the drill, when the drill proceeds to the rear end portion of the illumination lens to take out the illumination lens from the distal end portion.

The present invention has been made in view of the above circumstance. It is an object to provide an endoscope having a structure wherein the illumination lens in which the distal end face is exposed from the opening of the insertion portion distal end while the rear end face abuts on or comes close to the distal end face of the light guide can be replaced without damaging the light guide abutting on the illumination lens, and methods of producing and repairing the endoscope.

### MEANS FOR SOLVING PROBLEM

In order to achieve the object, an endoscope having the features of claim 1, an endoscope producing method having the features of claim 4 and a method of repairing an endoscope having the features of claim 5 are provided.

An endoscope of the invention includes an illumination lens which is fitted in a frame while a distal end face of the illumination lens is exposed, the frame being arranged in an insertion portion distal end, a bonded portion in an outer periphery of the illumination lens being fixed to an inner periphery of the frame with a bonding agent; and a light guide which is arranged in the frame while a distal end face of the light guide is orientated toward a rear end face of the illumination lens, the endoscope is characterized in that a non-bonding portion which prevents adhesion of the bonding agent is formed between the bonded portion of the illumination lens and the distal end face of the light guide in the frame.

An endoscope producing method of the invention is characterized by including forming a lens fitting circumferential surface in an inner periphery of a frame arranged in an insertion portion distal end; continuously forming a circumferential groove having a diameter larger than that of the lens fitting circumferential surface at the back of the lens fitting circumferential surface, the circumferential groove being located in the inner periphery of the frame; applying a tentative fixing bonding agent onto the lens fitting circumferential surface; fitting an illumination lens in the lens fitting circumferential surface of the frame such that a rear end portion is latched in a circumferential groove while a rear end face is orientated toward a distal end face of a light guide arranged in the circumferential groove in order to output illumination light; and fixing the illumination lens to the inner periphery of the frame by causing a real fixing bonding agent to penetrate between an outer periphery of the illumination lens tentatively fixed with the tentative fixing bonding agent and the lens fitting circumferential surface of the frame.

A method of the invention of repairing an endoscope having a frame in which a lens fitting circumferential surface and a circumferential groove are formed in an inner periphery, the circumferential groove being continuously formed at the back of the lens fitting circumferential surface while having a diameter larger than that of the lens fitting circumferential surface, an illumination lens being fixed to the lens fitting circumferential surface in the frame by a bonding agent in the endoscope, the endoscope repairing method is characterized by including cutting the illumination lens in a range of the lens fitting circumferential surface to removed the bonding agent located between the lens fitting circumferential surface and the illumination lens.

### EFFECT OF THE INVENTION

According to the present invention, in the endoscope and the methods of producing and repairing thereof, the illumination lens in which the distal end face is exposed from the opening of the insertion portion distal end while the rear end face abuts on or comes close to the distal end face of the light guide can be replaced without damaging the light guide abutting on the illumination lens.

### BRIEF DESCRIPTION OF DRAWINGS

FIG. 1 is a front view showing a schematic configuration of an endoscope according to a first embodiment of the invention;
FIG. 2 is a front view showing a distal end face of an insertion portion in the endoscope of FIG. 1;
FIG. 3 is a sectional view showing a schematic configuration of a distal end portion taken along line III-III of FIG. 2;
FIG. 4 is an enlarged sectional view showing a distal end side of a light guide unit of FIG. 3;
FIG. 5 is an enlarged sectional view showing fixing between an illumination lens and a frame in the light guide unit of FIG. 4;
FIG. 6 is an enlarged sectional view showing the fixing between the illumination lens and the frame when the illumination lens whose outer periphery abuts on an output end face of a light guide is used as the illumination lens of FIG. 5;
FIG. 7 is an enlarged sectional view showing a modification in the configuration of the light guide unit of FIG. 4;
FIG. 8 is an enlarged sectional view showing a distal end side of the light guide unit of an endoscope;
FIG. 9 is an enlarged sectional view showing the fixing between the illumination lens and the frame in the light guide unit of FIG. 8;
FIG. 10 is an enlarged sectional view showing the distal end side of the light guide unit of an endoscope;
FIG. 11 is an enlarged sectional view showing the fixing between the illumination lens and the frame in the light guide unit of FIG. 10;
FIG. 12 is an enlarged exploded sectional view showing the distal end side of the light guide unit of the endoscope; and
FIG. 13 is an enlarged sectional view showing the distal end side of the light guide unit of an endoscope.

### EXPLANATIONS OF LETTERS OR NUMERALS

- 1: Endoscope
- 3: Insertion portion
- 10: Distal end portion
- 33a: Illumination lens
- 33ac: Light guide
- 33ak: Plane of curvature
- 33b: Light guide
- 33bs: Output end face
- 33d: Frame
- 33dn: lens Fitting circumferential surface
- 33dm: Circumferential groove
- 70: Non-bonding portion
- 100: (real fixing) Bonding agent
- 133d: Frame
- 133dn: Lens fitting circumferential surface
- 133dp: Groove portion
- 170: Non-bonding portion
- 201: Endoscope
- 270: Non-bonding portion
- 301: Endoscope

### BEST MODE(S) FOR CARRYING OUT THE INVENTION

Exemplary embodiments of the invention will be described below with reference to the drawings. In the following embodiments, the endoscope in which a wide angle lens having a viewing angle of 140° or larger is arranged in an observation optical system is described by way of example.

### First Embodiment

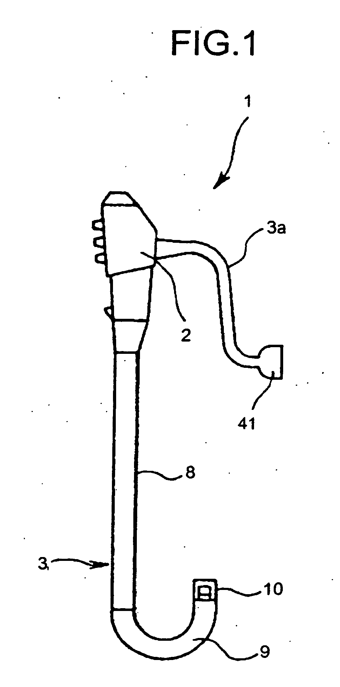

FIG. 1 is a front view showing a schematic configuration of an endoscope according to a first embodiment of the invention.
As shown in FIG. 1, an endoscope 1 includes an operation unit 2, an insertion portion 3, and a universal cord 3a. The operation unit 2 controls the bending operation and a channel system. The insertion portion 3 is inserted into the body cavity while the rear end side of the insertion portion 3 is connected to the operation unit 2. The universal cord 3a is extended from the operation unit 2, and the universal cord 3a has a connector unit 41 at the distal end thereof. The connector unit 41 is connected to a light source device (not shown) through a predetermined connector.

A tube 8, a bendable portion 9, and a distal end portion 10 are provided in the insertion portion 3. The tube 8 has flexibility, the bendable portion 9 is provided on the distal end side of the tube 8, and the distal end portion 10 is provided on the distal end side of the bendable portion 9. An imaging device (not shown) is incorporated in the distal end portion 10, and the imaging device takes an image of a test region, e.g., a region in the body cavity.

A bending operation knob (not shown) is arranged in the operation unit 2, and the bending operation knob remotely curves the bendable portion 9. The bending operation knob is operated to generate tensile action and releasing action in an operation wire (not shown) inserted through the insertion portion 3, which allows the bendable portion 9 to be bent in four directions.

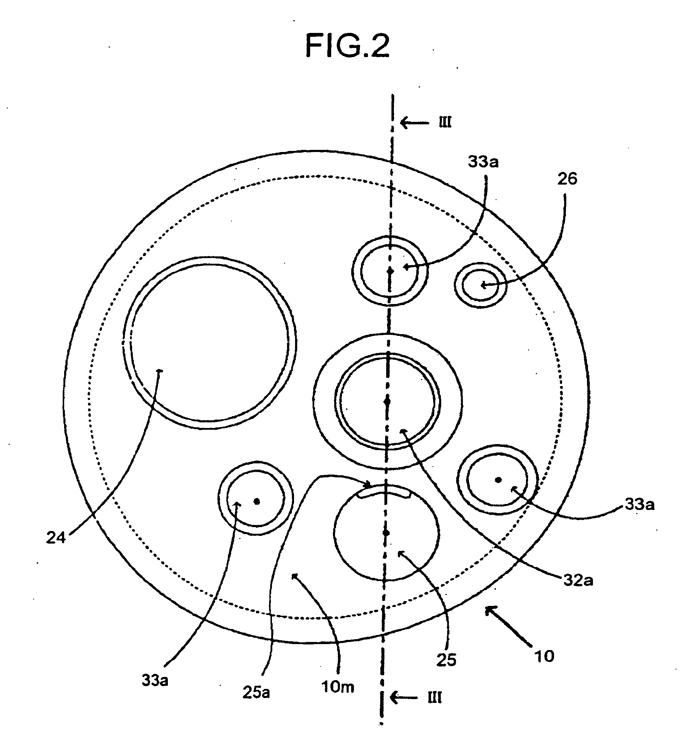

FIG. 2 is a front view showing a distal end face of the insertion portion in the endoscope of FIG. 1.
As shown in FIG. 2, an objective lens 32a, three illumination lenses 33a which are of the illumination optical system, an opening 24 for the treatment instrument or the like, an air supply and water supply nozzle 25, and a forward water-supply port 26 are arranged in a distal end face 10m of the distal end portion 10 of the endoscope insertion portion 3. The air supply and water supply nozzle 25 supplies air and water to clean dirt of the objective lens 32a or the three3 illumination lenses 33a when the distal end portion 10 is inserted into the body cavity. The forward water-supply port 26 cleans blood, mucus, and the like of a diseased part in the body cavity. Accordingly, plural openings are made in the distal end face 10m of the distal end portion 10 to arrange the objective lens 32a, the three illumination lenses 33a, the opening 24 for the treatment instrument or the like, the air supply and water supply nozzle 25, and the forward water-supply port 26.

The three illumination lenses 33a are arranged near a circumferential periphery portion of the objective lens 32a at predetermined angular intervals. The opening 24 for the treatment instrument or the like, the air supply and water supply nozzle 25, and the forward water-supply port 26 are arranged between the illumination lenses 33a and near the circumferential periphery portion of the objective lens 32a.

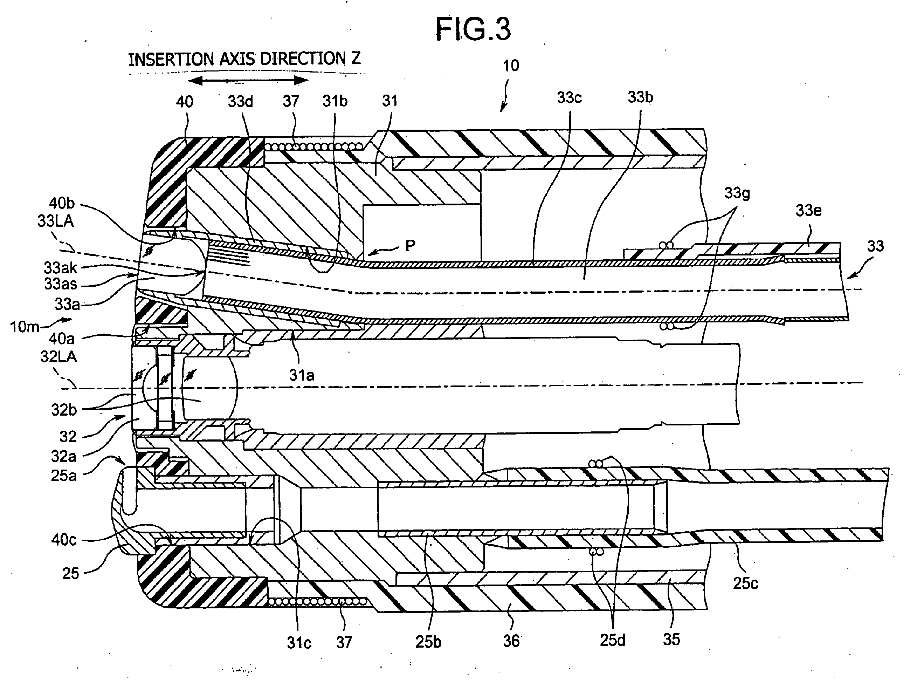

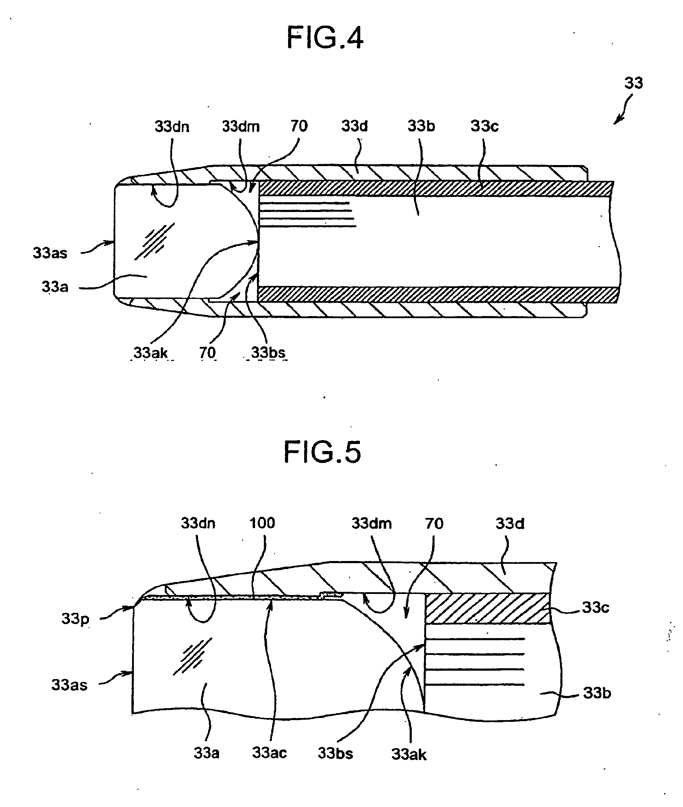

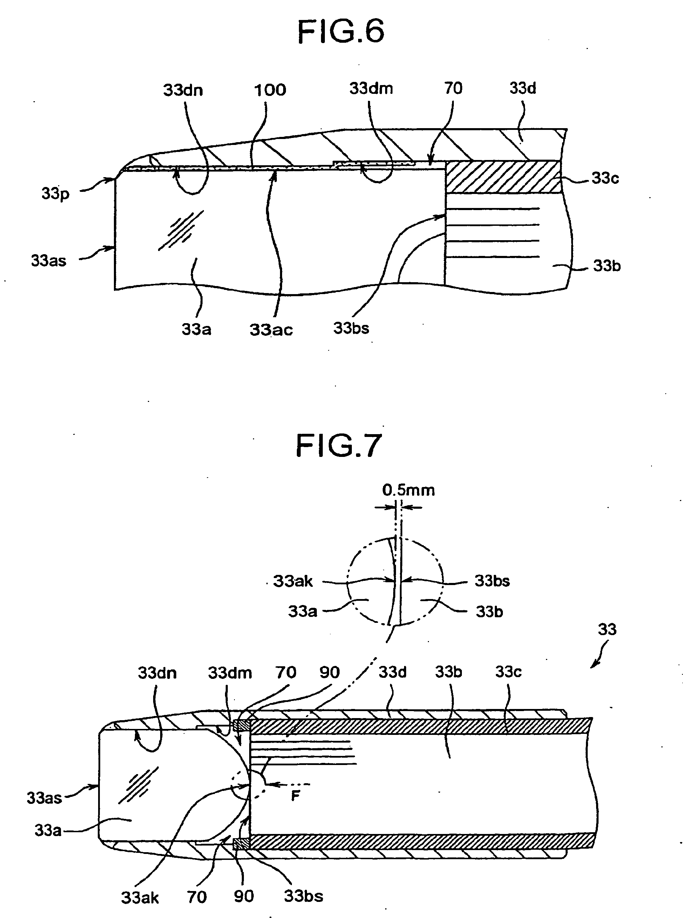

FIG. 3 is a sectional view showing a schematic configuration of the distal end portion taken along line III-III of FIG. 2, FIG. 4 is an enlarged sectional view showing the distal end side of the light guide unit of FIG. 3, FIG. 5 is an enlarged sectional view showing the fixing between the illumination lens and the frame in the light guide unit of FIG. 4, and FIG. 6 is an enlarged sectional view showing the fixing between the illumination lens and the frame when the illumination lens whose outer periphery abuts on the output end face of the light guide is used as the illumination lens of FIG. 5.

As shown in FIG. 3, a distal end rigid portion 31 is arranged in the distal end portion 10 of the endoscope 1. Long holes 31a, 31b, and 31c are made in the distal end rigid portion 31. An imaging unit 32, a light guide unit 33, and an air supply and water supply nozzle 25 are arranged in the long holes 31a, 31b, and 31c respectively.

The long holes 31a and 31c in which the imaging unit 32 and the air supply and water supply nozzle 25 are arranged are made in parallel with an insertion axis direction Z of the endoscope insertion portion 3. The long hole 31b in which the light guide unit 33 is arranged is made in the direction of an axis 33LA oblique to the direction of an axis 32LA in which an later-mentioned observation optical system 32b of the distal end rigid portion 31 is arranged. Actually three long holes 31b are made although not shown.

On the distal end side in the insertion axis direction Z of the distal end rigid portion 31, a cap 40 is topped to cover a front face and an outer periphery surface of the distal end rigid portion 31. Openings 40a, 40b, and 40c are formed in the cap 40. The imaging unit 32, the light guide unit 33, the air supply and water supply nozzle 25 and the like are arranged in the openings 40a, 40b, and 40c. The openings 40a, 40b, and 40c formed in the cap 40 correspond to the openings formed in the distal end face 10m of the distal end portion 10.

The openings 40a and 40c in which the imaging unit 32 and the air supply and water supply nozzle 25 are arranged are formed in parallel with the insertion axis direction Z. The opening 40b in which the light guide unit 33 is arranged is formed in the direction of the axis 33LA oblique to the direction of the axis 32LA of the cap 40. Although not shown, actually three openings 40b are formed so as to become equal to the number of long holes 31b.

The imaging unit 32 is inserted into the long hole 31a and the opening 40a in parallel to the insertion axis direction, and the imaging unit 32 is fixed to the long hole 31a and the opening 40a using a filler. In FIG. 3, the long hole 31a is made in the substantial center of the distal end rigid portion 31, and the opening 40a is formed in the substantial center of the cap 40.

The imaging unit 32 includes an observation optical system 32b and an imaging device (not shown). The observation optical system 32b is formed by the plural wide angle lenses, and the wide angle lens has an objective lens 32a having the wide viewing angle of 140° or larger. The imaging device such as CCD is provided on the rear end side of the observation optical system 32b.

The light guide unit 33 mainly includes a single illumination lens 33a and a light guide 33b. In the illumination lens 33a, the rear end face is formed in a plane of curvature 33ak projected toward the direction of the light guide 33b. The light guide 33b includes plural optical fibers provided on the rear end side of the illumination lens 33a. The three light guide units 33 are arranged so as to surround the imaging unit 32.

A metal pipe 33c which is of a hard pipe-shape member is coated on the outer periphery of the light guide 33b, and the outer periphery of the light guide 33b is fixed to the inside of the metal pipe 33c with the bonding agent or the like. As described above, the light guide 33b includes the plural optical fibers, and an output end face 33bs orientated toward the rear end face of the illumination lens 33a is polished. Although the light guide 33b includes the plural optical fibers in the first embodiment, sometimes a glass rod (not shown) or a lens (not shown) is arranged between the output end face 33bs and the rear end face of the illumination lens 33a. In this case, the light guide 33 including the glass rod or lens is referred to as light guide 33b, and the output end face of the glass rod or lens is referred to as output end face 33bs of light guide 33b.

The distal end side of the light guide 33b and the illumination lens 33a are fitted in and fixed to a frame 33d which is arranged in the long hole 31b of the distal end rigid portion 31 and in the opening 40b of the cap 40. Specifically, the frame 33d is formed in a substantially cylindrical shape in which the outer periphery of the distal end is tapered such that the opening of the distal end is decreased in diameter. As shown in FIGS. 4 and 5, the inner periphery of the frame 33d is formed by a lens fitting circumferential surface 33dn and a circumferential groove 33dm. The lens fitting circumferential surface 33dn has the diameter substantially equal to the diameter of the illumination lens 33a. The circumferential groove 33dm is formed at the back of the peripheral surface 33dn while continued in the insertion axis direction Z, and the circumferential groove 33dm has the diameter larger than the diameter of the lens fitting circumferential surface 33dn.

The distal end side of the light guide 33b coated with the metal pipe 33c is fitted in the circumferential groove 33dm, and the outer periphery on the distal end side is fixed to the circumferential groove 33dm with the bonding agent or the like.
As shown in FIG. 5, the illumination lens 33a is fitted in and fixed to the frame 33d. Specifically, a bonded portion 33ac of the outer periphery of the illumination lens 33a is bonded to the lens fitting circumferential surface 33dn with a bonding agent 100, and thereby the illumination lens 33a is fixed to the inside of the frame 33d.

A distal end face 33as of the illumination lens 33a is exposed outward from the distal end portion 10 of the insertion portion 3 through the opening 40b of the cap 40. The distal end face 33as is flush with the distal end face of the cap 40.

The plane of curvature 33ak of the illumination lens 33a abuts on the output end face 33bs of the light guide 33b. The plane of curvature 33ak of the illumination lens 33a may not abut on but come close to the output end face 33bs of the light guide 33b unless optical properties such as color shading are degraded.

A non-bonding portion 70 is formed between the bonded portion 33ac of the outer periphery of the illumination lens 33a and the output end face 33bs of the light guide 33b. The non-bonding portion 70 is a space provided between the outer periphery of the rear end portion of the illumination lens 33a and the inner periphery of the frame 33d, and the non-bonding portion 70 is formed by the circumferential groove 33dm provided in the inner periphery of the frame 33d in a mode in which the non-bonding portion 70 is separated from at least the outer periphery of the rear end portion of the illumination lens 33a. In the insertion axis direction Z of the non-bonding portion 70, there is a distance of 0.5 mm or larger between the bonded portion 33ac and the output end face 33bs of the light guide 33b.

A method of fitting and fixing the illumination lens 33a in and to the frame 33d will be described below. A tentative fixing bonding agent is applied to several points of the lens fitting circumferential surface 33dn of the frame 33d. Then, the illumination lens 33a is fitted in the lens fitting circumferential surface 33dn in the frame 33d such that the plane of curvature 33ak of the illumination lens 33a abuts on the output end face 33bs of the light guide 33b.

In the case where the light guide 33b is not previously fitted in the frame 33d, the illumination lens 33a is fitted in the lens fitting circumferential surface 33dn in the frame 33d to an assumed position where the plane of curvature 33ak abuts on the output end face 33bs of the light guide 33b.

Then, the real fixing bonding agent 100 having a regulated amount is applied to a gap 33p (see FIG. 5) between the outer periphery of the distal end of the illumination lens 33a and the inner periphery of the distal end of the frame 33d. Then, as shown in FIG. 5, the applied bonding agent 100 permeates and penetrates between the outer periphery of the illumination lens 33a and the lens fitting circumferential surface 33dn of the frame 33d.

A part of the bonding agent 100 penetrates to the circumferential groove 33dm of the frame 33d. At this point, because the non-bonding portion 70 has the relatively wide space, the bonding agent 100 penetrating to the circumferential groove 33dm does not adhere to the outer periphery of the illumination lens 33a in the non-bonding portion 70.

Finally, the bonding agent 100 is dried to fix the illumination lens 33a to the inside of the frame 33d by the bonding agent 100.

Returning to FIG. 3, the frame 33d is fixed to the distal end rigid portion 31 using a fixing screw or the like. The light guide 33b is extended rearward from the distal end rigid portion 31 and connected to an illumination device (not shown).

A part of the metal pipe 33c and the light guide 33b are covered with an outer covering tube 33e. The outer covering tube 33e is fixed to the outer periphery of the metal pipe 33c by a tied string 33g.

The metal pipe 33c is bent at a predetermined point P along the direction of the axis 32LA of the observation optical system 32b from the axis oblique to the direction of the axis 32LA as the optical axis in which the observation optical system 32b is arranged, i.e., the axis 33LA of the illumination lens 33a. As a result the light guide 33b is also bent at the predetermined point P along the bent shape of the metal pipe 33c.

Accordingly, the light guide unit 33 is arranged in the axis 33LA oblique to the direction of the axis 32LA in which the observation optical system 32b is arranged. Because the observation optical system 32b is formed by the lens having the wide viewing angle, it is necessary that the light guide unit 33 illuminating the body cavity evenly illuminate the body cavity over the wide range.

The air supply and water supply nozzle 25 is fitted in and fixed to the long hole 31c of the distal end rigid portion 31 and the opening 40c of the cap 40. For example, the air supply and water supply nozzle 25 is made of metal. An opening 25a is provided on the distal end side of the air supply and water supply nozzle 25.

The opening 25a is provided such that the water or air discharged from the air supply and water supply nozzle 25 is ejected through the opening 25a toward the direction which is parallel to a plane orthogonal to the optical axis 32LA of the imaging unit 32 and passes through both the surface of the objective lens 32a and the surface of the illumination lens 33a. The air supply and water supply nozzle 25 is formed at the position, where the air supply and water supply nozzle 25 is not included in the viewing angle range of the objective lens 32a, while projected from the distal end face 10m of the distal end portion 10 of the insertion portion 3.

The rear end side of the air supply and water supply nozzle 25 is formed in a pipe shape, and an air supply and water supply tube 25c is connected to the rear end side through a connecting pipe 25b. Therefore, a water supply channel is formed by the connecting pipe 25b and the air supply and water supply tube 25c. The air supply and water supply tube 25c is fixed to the connecting pipe 25b by a tied string 25d.

A part of the distal end rigid portion 31 is fixed to a part of a bending piece 35. The rear end side of the distal end rigid portion 31 and the bending piece 35are covered with an outer covering tube 36. The outer covering tube 36 is fixed to the distal end rigid portion 31 by a tied string 37.

Then, an endoscope repairing method which is the action of the endoscope 1 of the first embodiment having the above-described configuration will be described. Specifically, the illumination lens 33a replacement method will be described below.

A drill which is of the cutting tool is brought close to the distal end face 33as of the illumination lens 33a fixed to the inside of the frame 33d along the optical axis from the outside, and the drill is caused to proceed to the distal end of the circumferential groove 33dm, i.e., to the rear end of the bonded portion 33ac to cut the illumination lens. As a result, the bonding agent 100 of the bonded portion 33ac is removed in the illumination lens 33a by the cutting. This enables the fixing to be released between the illumination lens 33a and the frame 33d.

Finally, the distal end portion 10 of the insertion portion 3 of the endoscope 1 is inclined downward to take out the illumination lens, in which the fixing is released and a part is cut, from the frame 33d. Therefore, the illumination lenses 33a can be replaced. The method of fitting and fixing the new illumination lens 33a in and to the frame 33d is as described above.

Thus, in the endoscope 1 of the first embodiment, the circumferential groove 33dm having the diameter larger than that of the lens fitting circumferential surface 33dn is formed in the inner periphery of the frame 33d which the illumination lens 33a is fitted in and fixed to.

Conventionally, in replacing the illumination lenses 33a, when the drill as the cutting tool is brought close to the distal end face 33as along the optical axis from the outside to cut the illumination lens 33a, sometimes the output end face 33bs is damaged by the distal end of the drill, because the bonded portion 33ac of the illumination lens 33a and the output end face 33bs of the light guide 33b are close to each other while the circumferential groove 33dm does not exist between the bonded portion 33ac and the output end face 33bs.

On the contrary, in the first embodiment, because the circumferential groove 33dm having the diameter larger than that of the lens fitting circumferential surface 33dn is formed in the inner periphery of the frame 33d, the non-bonding portion 70 is formed between the bonded portion 33ac of the illumination lens 33a and the output end face 33bs of the light guide 33b. The non-bonding portion 70 has the distance of 0.5 mm or larger in the insertion axis direction Z, and the non-bonding portion 70 is formed by at least the circumferential groove 33dm. Therefore, the bonding agent 100 penetrating to the circumferential groove 33dm does not adhere to the outer periphery of the illumination lens 33a in the non-bonding portion 70, because the non-bonding portion 70 has the relatively wide space.

Accordingly, when the distal end of the drill is caused to proceed to the rear end of the bonded portion 33ac, the distal end of the drill does not come into contact with the output end face 33bs of the light guide 33b, so that the output end face 33bs is never damaged. This enables the illumination lenses 33a to be replaced without damaging the output end face 33bs of the light guide 33b.

The first embodiment is not limited to the case where the rear end face of the illumination lens 33a is formed as the plane of curvature 33ak projected toward the direction of the light guide 33b. For example, as shown in FIG. 6, the first embodiment can also be applied in the case where a part of a flat portion is formed in a concave shape with no plane of curvature 33ak in the rear end face of the illumination lens 33a while the flat portion abuts on the output end face 33bs of the light guide 33b.

Specifically, as shown in FIG. 6, because the circumferential groove 33dm having the diameter larger than that of the lens fitting circumferential surface 33dn is formed in the inner periphery of the frame 33d, even if a part of the flat portion is formed in the concave shape in the rear end face of the illumination lens 33a while the flat portion abuts on the output end face 33bs of the light guide 33b, the non-bonding portion 70 is formed between the bonded portion 33ac of the illumination lens 33a and the output end face 33bs of the light guide 33b. The non-bonding portion 70 has the distance of 0.5 mm or larger in the insertion axis direction Z and the non-bonding portion 70 is formed by at least the groove 33dm.

Because the non-bonding portion 70 has the relatively wide space, the bonding agent 100 penetrating into the circumferential groove 33dm does not adhere to the outer periphery of the illumination lens 33a in the non-bonding portion 70. Therefore, when the distal end of the drill is caused to proceed to the rear end of the bonded portion 33ac, the distal end of the drill does not come into contact with the output end face 33bs of the light guide 33b, so that the output end face 33bs is not damaged. Accordingly, the illumination lens 33a whose rear end of the outer periphery abuts on the output end face 33bs of the light guide 33b can be replaced with new one without damaging the output end face 33bs of the light guide 33b.

A modification will be described below. In the first embodiment, the endoscope in which the wide angle lens having the viewing angle of 140° or larger is arranged in the observation optical system 32b is described as an example of the endoscope 1. The invention is not limited to the first embodiment. Obviously the same effect can be obtained even if the invention is applied to an endoscope in which the lens having the usual viewing angle (140° or smaller) is used as the objective lens 32a and the observation optical system 32b.

Accordingly, the light guide unit 33 may not be arranged in the axis 33LA oblique to the direction of the axis 32LA in which the observation optical system 32b is arranged, but arranged in the direction of the axis parallel to the axis 32LA in which the observation optical system 32b is arranged.

In the first embodiment, the three illumination lenses 33a are arranged in the distal end portion. Alternatively, one illumination lens 33a or plural illumination lenses 33a may be arranged in the distal end portion. In this case, obviously the same number of light guide units 33 as the number of illumination lenses is provided.

In the first embodiment, the drill is cited as an example of the cutting tool used in cutting the illumination lens 33a. The invention is not limited to the drill, but a reamer may be used and obviously any cutting tool may be used as long as the cutting tool can cut the illumination lens 33a.

In the first embodiment, the plane of curvature 33ak of the illumination lens 33a abuts on the output end face 33bs which is of the distal end face of the light guide 33b. However, because a force F (see FIG. 7) for always pushing out the illumination lens 33a toward the distal end in the insertion direction is always applied to the illumination lens 33a from the light guide 33b, there is a possibility that the fixing between the illumination lens 33a and the frame 33d with bonding agent 100 is released by the force F.

As shown in FIG. 7, a thin ring-shape plate member 90 may fixedly be arranged at a position where the plane of curvature 33ak of the circumferential groove 33dm of the frame 33d and the output end face 33bs abut on each other. Therefore, the distal end face of the metal pipe 33c coating the light guide 33b abuts on the plate member 90.

Accordingly, although the plane of curvature 33ak of the illumination lens 33a and the output end face 33bs which is of the distal end face of the light guide 33b abuts on each other in the first embodiment, the plane of curvature 33ak and the output end face 33bs are arranged while separated from each other by the distance of 0.5 mm as shown in FIG. 7. The distance can be changed within the range with which the optical properties such as the color shading are not degraded.

Because the plate member 90 is fixed to the circumferential groove 33dm, even if the force F for always pushing out the plate member 90 toward the distal end of the insertion direction is applied to the plate member 90 from the light guide 33b, only the output end face 33bs or the distal end face of the metal pipe 33c covering the light guide 33b abuts on the plate member 90, and the output end face 33bs does not abut on the plane of curvature 33ak.

Therefore, the force F for always pushing out the illumination lens 33a toward the distal end of the insertion direction is not applied to the illumination lens 33a from the light guide 33b, which prevents the possibility that the fixing between the illumination lens 33a and the frame 33d with the bonding agent 100 is released by the force F.

Furthermore, the plane of curvature 33ak and the output end face 33bs can easily be separated from each other by the use of the plate member 90, compared with the case where the circumferential groove having the diameter larger than that of the circumferential groove 33dm is formed in the circumferential groove 33dm to separate the plane of curvature 33ak and the output end face 33bs from each other, and the use of the plate member 90 can secure the larger abutting area which the distal end face of the metal pipe 33c abuts on.

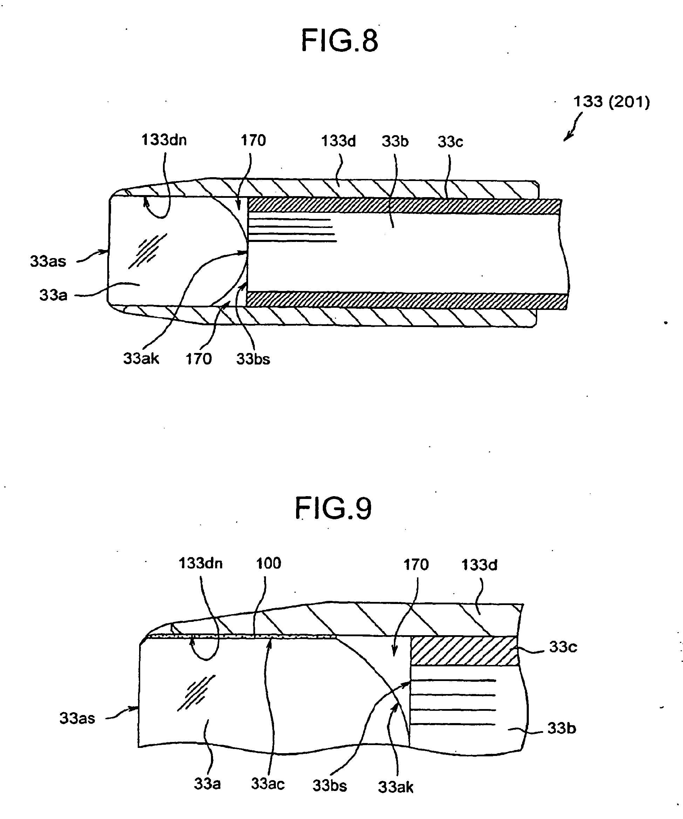

Further example FIG. 8 is an enlarged sectional view showing the distal end side of the light guide unit of an endoscope, and FIG. 9 is an enlarged sectional view showing the fixing between the illumination lens and the frame in the light guide unit of FIG. 8.

The configuration of an endoscope 201 of this example differs from that of the endoscope 1 shown in FIGS. 1 to 6 in that the circumferential groove is not formed in the inner periphery of the frame. Therefore, only the different points will be described, the same configuration as the first embodiment is designated by the same numeral, and the description will not be repeated.

As shown in FIG. 8, the distal end side of the light guide 33b and the illumination lens 33a are fitted in and fixed to a frame 133d which is arranged in the long hole 31b of the distal end rigid portion 31 and in the opening 40b of the cap 40.

Specifically, the frame 133d is formed in a substantially cylindrical shape in which the outer periphery of the distal end is tapered such that the opening of the distal end is decreased in diameter. As shown in FIGS. 8 and 9, the inner periphery of the frame 133d is formed by a lens fitting circumferential surface 133dn having the diameter substantially similar to the diameter of the illumination lens 33a.

The distal end side of the light guide 33b coated with the metal pipe 33c is fitted in the lens fitting circumferential surface 133dn, and the outer periphery on the distal end side is fixed to the lens fitting circumferential surface 133dn with the bonding agent or the like.
As shown in FIG. 8, the illumination lens 33a whose rear end face is formed in the plane of curvature 33ak projected toward the direction of the light guide 33b is fitted in and fixed to the frame 133d. Specifically, the bonded portion 33ac formed in a part of the outer periphery of the illumination lens 33a is bonded to the lens fitting circumferential surface 133dn with the bonding agent 100, and thereby the illumination lens 33a is fixed to the inside of the frame 133d.

The plane of curvature 33ak of the illumination lens 33a abuts on the output end face 33bs of the light guide 33b. The plane of curvature 33ak of the illumination lens 33a may not abut on but come close to the output end face 33bs of the light guide 33b unless the optical properties such as the color shading are degraded.

A non-bonding portion 170 is formed between the bonded portion 33ac of the outer periphery of the illumination lens 33a and the output end face 33bs of the light guide 33b. The non-bonding portion 170 is formed by a space between the plane of curvature 33ak and the inner periphery (lens fitting circumferential surface 133dn) of the frame 133d, and the plane of curvature 33ak is formed by projecting the rear end face of the illumination lens 33a toward the direction of the light guide 33b. In the insertion axis direction Z of the non-bonding portion 170, there is the distance of 0.5 mm or larger between the bonded portion 33ac and the output end face 33bs of the light guide 33b. Thus, the non-bonding portion 170 has the distance of 0.5 mm or larger between the bonded portion 33ac and the output end face 33bs of the light guide 33b by a curvature of the plane of curvature 33ak provided in the rear end face of the illumination lens 33a.

Then, an endoscope repairing method which is the action of the endoscope 201 having the above-described configuration will be described. Specifically, the illumination lens 33a replacement method will be described below.

The drill which is of the cutting tool is brought close to the distal end face 33as of the illumination lens 33a fixed to the inside of the frame 133d along the optical axis from the outside, and the drill is caused to proceed to the rear end of the illumination lens 33a, i.e., to the rear end of the bonded portion 33ac to cut the illumination lens. As a result, the bonding agent 100 of the bonded portion 33ac is removed in the illumination lens 33a by the cutting. This enables the fixing to be released between the illumination lens 33a and the frame 133d.

Finally, the distal end portion 10 of the insertion portion 3 of the endoscope 201 is inclined downward to take out the illumination lens, in which the fixing is released and a part is cut, from the frame 33d. Therefore, the illumination lenses 33a can be replaced.

Thus, in the endoscope 201, the rear end face of the illumination lens 33a is formed as the plane of curvature 33ak projected toward the direction of the light guide 33b, so that the non-bonding portion 170 having the distance of 0.5 mm or larger in the insertion axis direction Z is formed between the bonded portion 33ac of the illumination lens 33a and the output end face 33bs of the light guide 33b by the curvature of the plane of curvature 33ak. As a result, when the distal end of the drill is caused to proceed to the rear end of the bonded portion 33ac in the replacement, the distal end of the drill does not come into contact with the output end face 33bs of the light guide 33b.

Accordingly, the output end face 33bs is not damaged, so that the illumination lenses 33a can be replaced without damaging the output end face 33bs of the light guide 33b.

A modification will be described below. The same effect can also be obtained even if this example is applied to an endoscope in which the lens having the usual viewing angle (140° or smaller) is used as the observation optical system 32b.

The three illumination lenses 33a are arranged in the distal end portion. Alternatively, one illumination lens 33a or plural illumination lenses 33a may be arranged in the distal end portion. In this case, obviously the same number of light guide units 33 as the number of illumination lenses is provided.

The thin ring-shape plate member 90 shown in FIG. 7 may fixedly be arranged at the position where the plane of curvature 33ak of the lens fitting circumferential surface 133dn of the frame 133d and the output end face 33bs abut on each other.

The, drill is cited as an example of the cutting tool used in cutting the illumination lens 33a.
Alternatively, a reamer may be used and obviously any cutting tool may be used as long as the cutting tool can cut the illumination lens 33a.

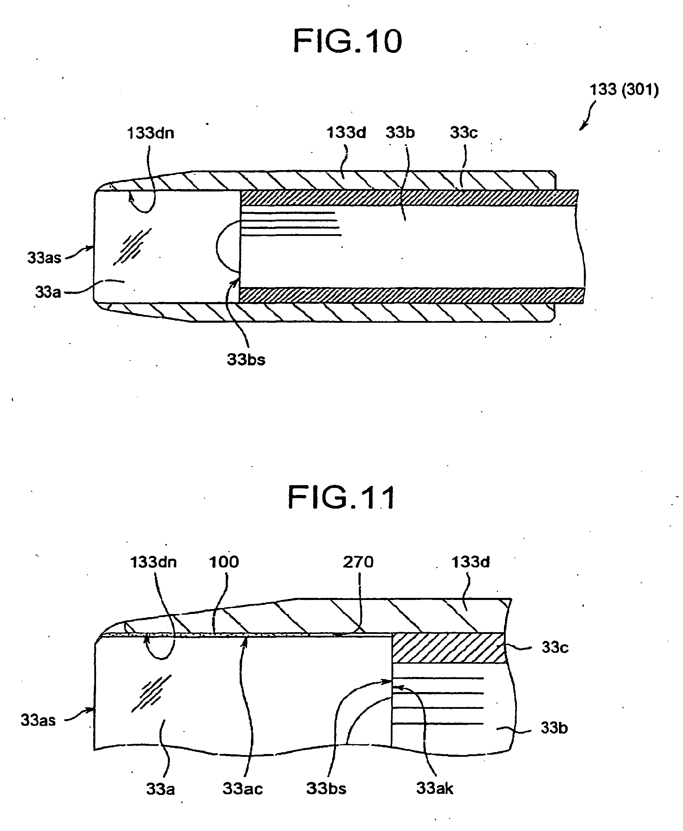

Further example FIG. 10 is an enlarged sectional view showing the distal end side of the light guide unit of an endoscope, and FIG. 11 is an enlarged sectional view showing the fixing between the illumination lens and the frame in the light guide unit of FIG. 10.

The configuration of an endoscope 301 differs from that of the endoscope 201 shown in FIGS. 8 and 9 in that an amount of bonding agent 100 penetrating between the outer periphery of the illumination lens 33a and the lens fitting circumferential surface 133dn of the frame 133d is adjusted. Therefore, only the different points will be described, the same configuration as the previous example is designated by the same numeral, and the description will not be repeated.

As shown in FIG. 10, the illumination lens 33a whose rear end of the outer periphery abuts on the output end face 33bs of the light guide 33b is fitted in and fixed to the inside of the frame 133d. Specifically, as shown in FIG. 11, the bonded portion 33ac in the outer periphery of the illumination lens 33a is bonded to the lens fitting circumferential surface 133dn with the amount of bonding agent 100 (hereinafter referred to as non-abutting amount) having an extent in which the bonding agent 100 does not penetrate to the rear end of the outer periphery of the illumination lens 33a, and thereby the illumination lens 33a is fixed to the inside of the frame 133d.

A non-bonding portion 270 is formed between the bonded portion 33ac of the illumination lens 33a and the output end face 33bs of the light guide 33b. The non-bonding portion 270 is provided between the outer periphery of the rear end portion of the illumination lens 33a and the inner periphery of the frame 133d which faces the outer periphery, and the non-bonding portion 270 is formed in a non-bonded area which suppresses the penetration of the bonding agent 100. Specifically, the non-bonding portion 270 which is of the non-bonded area is formed by the penetration of the bonding agent 100 having the non-abutting amount from the gap 33p (see FIG. 5) between the outer periphery of the distal end of the illumination lens 33a and the inner periphery of the distal end of the frame 33d to the gap between the outer periphery of the illumination lens 33a and the lens fitting circumferential surface 33dn of the frame 33d. That is, the space is formed between the lens fitting circumferential surface 133dn and the outer periphery of the illumination lens 33a. In the insertion axis direction Z of the non-bonding portion 270, there is the distance of 0.5 mm or larger between the bonded portion 33ac and the output end face 33bs of the light guide 33b. Thus, both the bonded portion 33ac fixed to the inside of the frame 33d with the bonding agent 100 and the non-bonding portion 270 not fixed to the frame 33d with the bonding agent 100 are formed respectively on the distal end face side and the read end face side in the outer periphery of the illumination lens 33a.

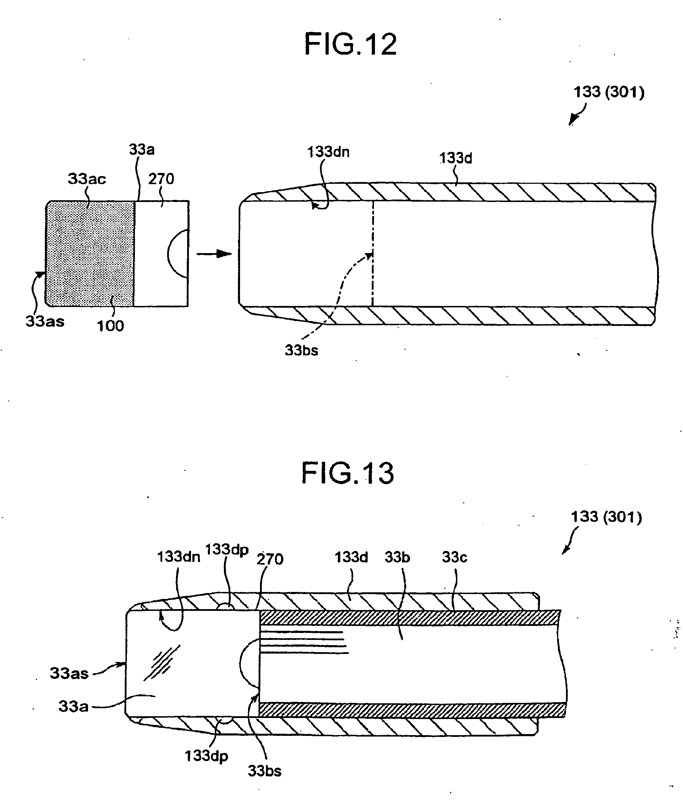

In forming the non-bonding portion 270 as the non-bonded area, as shown in FIG. 12, the bonding agent 100 is applied to the region of the bonded portion 33ac of the illumination lens 33a except for the region of the non-bonding portion 270 before the illumination lens 33a is fitted in the frame 133d, and the illumination lens 33a may be fitted in the frame 133d.

When the non-bonding portion 270 is formed as the non-bonded area, in order to suppress the penetration of the bonding agent 100, a coating (not shown) containing a stripping agent for stripping the bonding agent 100 may be performed to the outer periphery of the rear end portion of the illumination lens 33a and/or the inner periphery of the frame 133d which faces the outer periphery of the rear end portion of the illumination lens 33a.

Then, an endoscope repairing method which is the action of the endoscope 301 having the above-described configuration will be described. Specifically, the illumination lens 33a replacement method will be described below.

The drill which is of the cutting tool is brought close to the distal end face 33as of the illumination lens 33a fixed to the inside of the frame 133d along the optical axis from the outside, and the drill is caused to proceed to the rear end of the bonded portion 33ac in the outer periphery of the illumination lens 33a to cut the illumination lens. As a result, the bonding agent 100 having the non-abutting amount is removed by the cutting. This enables the fixing to be released between the illumination lens 33a and the frame 133d.

Finally, the distal end portion 10 of the insertion portion 3 of the endoscope 1 is inclined downward to take out the illumination lens 33a, in which the fixing is released and a part is cut, from the frame 133d. Therefore, the illumination lenses 33a can be replaced.

Thus, in the endoscope 301, the bonded portion 33ac in the outer periphery of the illumination lens 33a is fixed to the lens fitting circumferential surface 133dn with the bonding agent 100 having the non-abutting amount.

The non-bonding portion 270 having the distance of 0.5 mm or larger in the insertion axis direction Z is formed between the bonded portion 33ac of the illumination lens 33a and the output end face 33bs of the light guide 33b. When the distal end of the drill is caused to proceed to the rear end of the bonded portion 33ac in the replacement, the distal end of the drill does not come into contact with the output end face 33bs of the light guide 33b.

Accordingly, the output end face 33bs is not damaged, so that the illumination lenses 33a can be replaced without damaging the output end face 33bs of the light guide 33b.

A modification will be described below. The illumination lens 33a in which the rear end of the outer periphery abuts on the output end face 33bs of the light guide 33b is illustrated by way of example. The same effect can be also obtained even if the illumination lens 33a in which the rear end face is formed in the plane of curvature 33ak projected toward the direction of the light guide 33b is used.

Obviously the same effect can also be obtained even if the previous example is applied to an endoscope in which the lens having the usual viewing angle (140° or smaller) is used as the observation optical system 32b.

The three illumination lenses 33a are arranged in the distal end portion. Alternatively, one illumination lens 33a or plural illumination lenses 33a may be arranged in the distal end portion. In this case, obviously the same number of light guide units 33 as the number of illumination lenses is provided.

The thin ring-shape plate member 90 shown in FIG. 7 may fixedly be arranged at the position where the rear end face of the lens fitting circumferential surface 133dn of the frame 133d and the output end face 33bs abut on each other.

The drill is cited as an example of the cutting tool used in cutting the illumination lens 33a. However, the reamer may be used and obviously any cutting tool may be used as long as the cutting tool can cut the illumination lens 33a.

Further example FIG. 13 is an enlarged sectional view showing the distal end side of the light guide unit of an endoscope.

The configuration of an endoscope 301 differs from that of the endoscope 201 shown in FIGS. 8 and 9 in that groove portion is provided. Therefore, only the different points will be described, the same configuration is designated by the same numeral, and the description will not be repeated.

As shown in FIG. 13, the illumination lens 33a whose rear end of the outer periphery abuts on the output end face 33bs of the light guide 33b is fitted in and fixed to the inside of the frame 133d. Specifically, the bonded portion 33ac in the outer periphery of the illumination lens 33a is bonded to the lens fitting circumferential surface 133dn with the bonding agent 100, and thereby the illumination lens 33a is fixed to the inside of the frame 133d.

The non-bonding portion 270 is formed between the bonded portion 33ac of the illumination lens 33a and the output end face 33bs of the light guide 33b. The non-bonding portion 270 is a space provided between the outer periphery of the rear end of the illumination lens 33a and the inner periphery of the frame 133d. The non-bonding portion 270 is formed by a groove portion 133dp in the inner periphery of the frame 133d in the mode in which the groove portion 133dp is separated from the outer periphery of the rear end portion of the illumination lens 33a, and the groove portion 133dp has the diameter larger than that of the lens fitting circumferential surface 33dn. That is, the groove portion 133dp reserves the bonding agent 100 which penetrates from the gap 33p (see FIG. 5) between the outer periphery of the distal end of the illumination lens 33a and the inner periphery of the distal end of the frame 33d to the gap between the outer periphery of the illumination lens 33a and the lens fitting circumferential surface 33dn of the frame 33d, and the groove portion 133dp suppresses the penetration of the bonding agent 100 onto the rear side of the groove portion 133dp. As a result, the non-bonding portion 270 is formed between the bonded portion 33ac of the illumination lens 33a and the output end face 33bs of the light guide 33b. In the insertion axis direction Z of the non-bonding portion 270, there is the distance of 0.5 mm or larger between the bonded portion 33ac (groove portion 133dp) and the output end face 33bs of the light guide 33b.

Then, an endoscope repairing method which is the action of the endoscope 301 having the above-described configuration will be described. Specifically, the illumination lens 33a replacement method will be described below.

The drill which is of the cutting tool is brought close to the distal end face 33as of the illumination lens 33a fixed to the inside of the frame 133d along the optical axis from the outside, and the drill is caused to proceed to the rear end of the bonded portion 33ac in the outer periphery of the illumination lens 33a to cut the illumination lens. As a result, the bonding agent 100 having the non-abutting amount is removed by the cutting. This enables the fixing to be released between the illumination lens 33a and the frame 133d.

Finally, the distal end portion 10 of the insertion portion 3 of the endoscope 301 is inclined downward to take out the illumination lens 33a, in which the fixing is released and a part is cut, from the frame 133d.

Thus, in the endoscope 301, the groove portion 133dp having the diameter larger than that of the lens fitting circumferential surface 133dn is formed in the inner periphery of the frame 133d which the illumination lens 33a is fitted in and fixed to. The non-bonding portion 270 having the distance of 0.5 mm or larger in the insertion axis direction Z is formed by the groove portion 133dp between the bonded portion 33ac of the illumination lens 33a and the output end face 33bs of the light guide 33b. When the distal end of the drill is caused to proceed to the rear end of the bonded portion 33ac in the replacement, the distal end of the drill does not come into contact with the output end face 33bs of the light guide 33b.

Accordingly, the output end face 33bs is not damaged, so that the illumination lenses 33a can be replaced without damaging the output end face 33bs of the light guide 33b.

A modification will be described below. The illumination lens 33a in which the rear end of the outer periphery abuts on the output end face 33bs of the light guide 33b is illustrated by way of example. The example is not limited to the illumination lens 33a, but obviously the same effect can be also obtained even if the illumination lens 33a in which the rear end face is formed in the plane of curvature 33ak projected toward the direction of the light guide 33b is used.

Obviously the same effect can also be obtained even if the arrangement is applied to an endoscope in which the lens having the usual viewing angle (140° or smaller) is used as the observation optical system 32b.

The three illumination lenses 33a are arranged in the distal end portion. Alternatively, one illumination lens 33a or plural illumination lenses 33a may be arranged in the distal end portion. In this case, obviously the same number of light guide units 133 as the number of illumination lenses is provided.

The thin ring-shape plate member 90 shown in FIG. 7 may fixedly be arranged at the position where the rear end face of the lens fitting circumferential surface 133dn of the frame 133d and the output end face 33bs abut on each other.

The drill is cited as an example of the cutting tool used in cutting the illumination lens 33a. The example is not limited to the drill, but the reamer may be used and obviously any cutting tool may be used as long as the cutting tool can cut the illumination lens 33a.

### INDUSTRIAL APPLICABILITY

The present invention is useful as the endoscope having the illumination lens, used in illuminating the test region, at the distal end of the insertion portion. Particularly, in the case where the distal end face of the illumination lens is exposed from the opening of the insertion portion distal end while the rear end face abuts on or comes close to the distal end face of the light guide, the invention is suitable for replacing the illumination lenses without damaging the light guide abutting on the illumination lens.

## Claims

1. An endoscope (1; 201; 301) comprising:
an illumination lens (33a) which is fitted in a lens fitting circumferential surface (33dn; 133dn) formed on an inner periphery of a frame (33d; 133d) while a distal end face (33as) of the illumination lens (33a) is exposed, the frame (33d; 133d) being arranged in an insertion portion distal end of the endoscope, a bonded portion (33ac) in an outer periphery of the illumination lens (33a) being fixed to the lens fitting circumferential surface (33dn; 133dn) of the frame (33d; 133d) with a bonding agent (100); and
a light guide (33b) which is arranged in the frame (33d; 133d) while a distal end face of the light guide (33b) is orientated toward a proximal end face of the illumination lens (33a); **characterized by**
a non bonding portion (70; 170; 270) which is adapted to prevent adhesion of the bonding agent (100) between the bonded portion (33ac) of the illumination lens (33a) and the distal end face of the light guide (33b), the non bonding portion (70; 170; 270) being formed by a circumferential groove (33dm) which is successively formed at the proximal end of the lens fitting circumferential surface (33dn; 133dn) and which has a diameter larger than that of the lens fitting circumferential surface (33dn; 133dn).

2. The endoscope (1) according to claim 1, wherein the circumferential groove (33dm) is provided in the inner periphery of the frame (33d) in a mode in which a space is formed between the outer periphery of a rear end portion of the illumination lens (33a) and the inner periphery of the frame, and the light guide (33b) is fitted in the circumferential groove (33dm).

3. The endoscope (1; 201) according to claim 1, further comprising a ringshaped plate member (90) which abuts on the circumferential groove (33dm) to prevent the rear end face of the illumination lens (33a) from coming into contact with the light guide (33b).

4. An endoscope (1) producing method comprising:
forming a lens fitting circumferential surface (33dn) in an inner periphery of a frame (33d) arranged in an insertion portion distal end of an endoscope;
continuously forming a circumferential groove (33dm) having a diameter larger than that of the lens fitting circumferential surface (33dn) at the proximal end of the
lens fitting circumferential surface (33dn), the circumferential groove (33dm) being located in the inner periphery of the frame (33d);
applying a tentative fixing bonding agent onto the lens fitting circumferential surface (33dn);
fitting an illumination lens (33a) in the lens fitting circumferential surface (33dn) of the frame (33d) such that a proximal end portion of the illumination lens abuts on a distal end face of a light guide (33b) arranged in the circumferential groove (33dm) in order to output illumination light; and
fixing the illumination lens (33a) to the inner periphery of the frame (33d) by causing a real fixing bonding agent (100) to penetrate between an outer periphery of the illumination lens (33a) tentatively fixed with the tentative fixing bonding agent and the lens fitting circumferential surface (33dn) of the frame (33d).

5. A method of repairing the endoscope (1) according to any one of claims 1 to 3 or the endoscope (1) that has been produced with the method of claim 4,
the method comprising cutting the illumination lens in a range of the lens fitting circumferential surface (33dn) to remove the bonding agent (100) located between the lens fitting circumferential surface (33dn) and the illumination lens (33a).

## Patentansprüche

1. Endoskop (1; 201; 301) mit:
einer Beleuchtungslinse (33a), die in eine Linsenbefestigungs-Umfangsfläche (33dn, 133dn) eingepasst ist, welche an einem Innenumfang eines Rahmens (33d; 133d) ausgebildet ist, während eine distale Endfläche (33as) der Beleuchtungslinse (33a) freiliegt, wobei der Rahmen (33d; 133d) in einem distalen Ende des Einführbereichs des Endoskops angeordnet ist, ein geklebter Bereich (33ac) an einem Außenumfang der Beleuchtungslinse (33a) mit einem Haftmittel (100) an der Linsenbefestigungs-Umfangsfläche (33dn; 133dn) des Rahmens (33d; 133d) befestigt ist; und
einem Lichtleiter (33b), der in dem Rahmen (33d; 133d) angeordnet ist, während eine distale Endfläche des Lichtleiters (33b) zu einer proximalen Endfläche der Beleuchtungslinse (33a) ausgerichtet ist, **gekennzeichnet durch**
einen klebefreien Bereich (70; 170; 270), der ein Haften des Haftmittels (100) zwischen dem geklebten Bereich (33ac) der Beleuchtungslinse (33a) und der distalen Endfläche des Lichtleiters (33b) zu verhindern vermag, wobei der klebefreie Bereich (70; 170; 270) **durch** eine Umfangsnut (33dm) ausgebildet ist, welche nachfolgend am proximalen Ende der Linsenbefestigungs-Umfangsfläche (33dn; 133dn) ausgebildet ist und deren Durchmesser größer als derjenige der Linsenbefestigungs-Umfangsfläche (33dn; 133dn) ist.

2. Endoskop (1) nach Anspruch 1, wobei die Umfangsnut (33dm) im Innenumfang des Rahmens (33d) in einer Art und Weise vorgesehen ist, bei der zwischen dem Außenumfang des hinteren Endbereichs der Beleuchtungslinse (33a) und dem Innenumfang des Rahmens ein Raum ausgebildet ist, und der Lichtleiter (33b) in die Umfangsnut (33dm) eingepasst ist.

3. Endoskop (1; 201) nach Anspruch 1, das ferner ein ringförmiges Plattenelement (90) umfasst, welches an der Umfangsnut (33dm) anliegt, um zu verhindern, dass die hintere Endfläche der Beleuchtungslinse (33a) mit dem Lichtleiter (33b) in Kontakt kommt.

4. Verfahren zum Herstellen eines Endoskops (1), das umfasst:
Ausbilden einer Linsenbefestigungs-Umfangsfläche (33dn) in einem Innenumfang eines Rahmens (33d), der in einem distalen Ende des Einführbereichs eines Endoskops angeordnet ist;
kontinuierliches Ausbilden einer Umfangsnut (33dm), die einen Durchmesser besitzt, der größer als derjenige der Linsenbefestigungs-Umfangsfläche (33dn) am proximalen Ende der Linsenbefestigungs-Umfangsfläche (33dn) ist, wobei die Umfangsnut (33dm) im Innenumfang des Rahmens (33d) angeordnet ist;
Auftragen eines Haftmittels zum vorläufigen Befestigen auf die Linsenbefestigungs-Umfangsfläche (33dn);
Einpassen einer Beleuchtungslinse (33a) in die Linsenbefestigungs-Umfangsfläche (33dn) des Rahmens (33d) derart, dass ein proximaler Endbereich der Beleuchtungslinse an einer distalen Endfläche eines Lichtleiters (33b) anliegt, der in der Umfangsnut (33dm) angeordnet ist, um Beleuchtungslicht auszusenden; und
Befestigen der Beleuchtungslinse (33a) am Innenumfang des Rahmens (33d), indem ein Haftmittel (100) zum tatsächlichen Befestigen dazu gebracht wird, zwischen einen Außenumfang der Beleuchtungslinse (33a), die mit dem Haftmittel zum vorläufigen Befestigen provisorisch befestigt ist, und die Linsenbefestigungs-Umfangsfiläche (33dn) des Rahmens (33d) einzudringen.

5. Verfahren zum Reparieren des Endoskops (1) nach einem der Ansprüche 1 bis 3 oder des mit dem Verfahren nach Anspruch 4 hergestellten Endoskops (1),
wobei das Verfahren das Schneiden der Beleuchtungslinse in einem Bereich der Linsenbefestigungs-Umfangsfläche (33dn) umfasst, um das zwischen der Linsenbefestigungs-Umfangsfläche (33dn) und der Beleuchtungslinse (33a) vorhandene Haftmittel (100) zu entfernen.

## Revendications

1. Endoscope (1 ; 201 ; 301) comprenant :
une lentille d'éclairage (33a) qui est ajustée dans une surface circonférentielle d'ajustement de lentille (33dn ; 133dn) formée sur une périphérie interne d'une monture (33d ; 133d) alors qu'une face d'extrémité distale (33as) de la lentille d'éclairage (33a) est exposée, la monture (33d ; 133d) étant disposée dans une extrémité distale de la partie d'insertion de l'endoscope, une partie liée (33ac) dans une périphérie externe de la lentille d'éclairage (33a) étant fixée à la surface circonférentielle d'ajustement de lentille (33dn ; 133dn) de la monture (33d ; 133d) avec un agent de liaison (100) ; et
un guide de lumière (33b) qui est disposé dans la monture (33d ; 133d) alors qu'une face d'extrémité distale du guide de lumière (33b) est orientée vers une face d'extrémité proximale de la lentille d'éclairage (33a) ; **caractérisé par**
une partie non de liaison (70 ; 170 ; 270) qui est adaptée pour empêcher l'adhérence de l'agent de liaison (100) entre la partie liée (33ac) de la lentille d'éclairage (33a) et la face d'extrémité distale du guide de lumière (33b), la partie non de liaison (70 ; 170 ; 270) étant formée par une rainure circonférentielle (33dm) qui est successivement formée au niveau de l'extrémité proximale de la surface circonférentielle d'ajustement de lentille (33dn ; 133dn) et qui présente un diamètre supérieur à celui de la surface circonférentielle d'ajustement de lentille (33dn ; 133dn).

2. Endoscope (1) selon la revendication 1, dans lequel la rainure circonférentielle (33dm) est prévue dans la périphérie interne de la monture (33d) dans un mode dans lequel un espace est formé entre la périphérie externe d'une partie d'extrémité arrière de la lentille d'éclairage (33a) et la périphérie interne de la monture, et le guide de lumière (33b) est ajusté dans la rainure circonférentielle (33dm).

3. Endoscope (1 ; 201) selon la revendication 1, comprenant en outre un élément de plaque en forme de bague (90) qui vient en butée sur la rainure circonférentielle (33dm) pour empêcher la face d'extrémité arrière de la lentille d'éclairage (33a) de venir en contact avec le guide de lumière (33b).

4. Procédé de production d'un endoscope (1) comprenant les étapes consistant à :
former une surface circonférentielle d'ajustement de lentille (33dn) dans une périphérie interne d'une monture (33d) disposée dans une extrémité distale de la partie d'insertion d'un endoscope ;
former de manière continue une rainure circonférentielle (33dm) présentant un diamètre supérieur à celui de la surface circonférentielle d'ajustement de lentille (33dn) au niveau de l'extrémité proximale de la surface circonférentielle d'ajustement de lentille (33dn), la rainure circonférentielle (33dm) étant placée dans la périphérie interne de la monture (33d) ;
appliquer un agent de liaison de fixation temporaire sur la surface circonférentielle d'ajustement de lentille (33dn) ;
ajuster une lentille d'éclairage (33a) dans la surface circonférentielle d'ajustement de lentille (33dn) de la monture (33d) d'une manière telle qu'une partie d'extrémité proximale de la lentille d'éclairage vient en butée sur une face d'extrémité distale d'un guide de lumière (33b) disposé dans la rainure circonférentielle (33dm) afin de délivrer en sortie la lumière d'éclairage ; et
fixer la lentille d'éclairage (33a) sur la périphérie interne de la monture (33d) en amenant un agent de liaison de fixation réelle (100) à pénétrer entre une périphérie externe de la lentille d'éclairage (33a) fixée temporairement avec l'agent de liaison de fixation temporaire et la surface circonférentielle d'ajustement de lentille (33dn) de la monture (33d).

5. Procédé de réparation de l'endoscope (1) selon l'une quelconque des revendications 1 à 3 ou de l'endoscope (1) qui a été produit avec le procédé de la revendication 4,
le procédé comprenant la coupe de la lentille d'éclairage dans une plage de la surface circonférentielle d'ajustement de lentille (33dn) pour enlever l'agent de liaison (100) placé entre la surface circonférentielle d'ajustement de lentille (33dn) et la lentille d'éclairage (33a).
